# EUROPEAN PATENT SPECIFICATION

(11) **EP 0 393 003 B1**
(45) Date of publication and mention of the grant of the patent: **26.05.1993**
(21) Application number: 90870044.6
(22) Date of filing: 23.03.1990
(51) Int. Cl.: A61F 13/04

(54) **Composite material for medical or paramedical, particularly orthopaedic use**
Verbundmaterial für medizinische oder paramedizinische vorzugsweise orthopädische Anwendungen
Matériau composite pour usage médical ou paramédical, en particulier orthopédique

(30) Priority: 12.04.1989 US 338505
(43) Date of publication of application: 17.10.1990
(73) Proprietor: Ponnet, Tom Paul Marthe Ghislain, 2600 Berchem (BE)
(72) Inventor: Ponnet, Tom Paul Marthe Ghislain, 2600 Berchem (BE)
(74) Representative: Debrabandere, René

(56) References cited:
- EP-A- 0 263 552
- WO-A-88/09158
- JP-A-57 198 780
- US-A- 4 019 505

## Description

The invention relates to a composite material for medical or paramedical, particulary orthopaedic use, which comprises a core of a thermoplastic composition containing polycaprolactone and polyurethane and having a thickness between 0.05 and 25 mm, and on both sides thereof a coating of a soft resilient open cell foam plastic with a thickness outside the core between 0.05 and 1.5 mm.

Such composition is known from EP-A-O 263.552 in the name of the applicant. Said polycaprolactone-polyurethane composition is manufactured in the shape of plates or sheets, and is being used as such to replace plaster bandages.

The coatings may be partly penetrated in the core but their thickness outside the core lies between 0.05 and 1.5 mm. The plastic foam forms an insulating layer which avoids the weakened thermoplastic composition contacting directly the skin, delays the composition cooling and lets the skin breathe. The foam plastic outside the core is, before the application, in no way filled with thermoplastic material as for example the foam plastic from the composite material according to US-A 3,728,206 which foam plastic does not form a coating. The foam plastic forms a coating which prevents portions of the composite material sticking inadvertenly to one another. On the one hand, by exerting a small pression overlapping portions of the weakened thermoplastic composition may stick to one another through the foam plastic coating as the composition squeezes through the plastic foam, but on the other hand due to the presence of the plastic foam, such portions sticking to one another may still be released when or as long as the composition is in weakened condition.

Gripping the material with bare hands can be done. After some time and certainly after the thermoplastic material has set, the portions adhere very strongly to one another.

Layers of plastic foam with a thickness outside of the core of more than 1.5 mm would not let through the thermoplastic core material and prevent a good adherence between overlapping parts of the composite material.

The composition is made soft and self-adhesive by heating same above 60 degrees Celsius e.g. by dipping into hot water, and thereafter is put in the desired shape on the body. Parts of the material are stuck to one another. After cooling, the material forms a rigid unit.

The aim of the invention is to provide a composite material of the above mentionned kind with a reduced heating up time before it can be applied.

For this purpose the core of thermoplastic material is comprised of three layers, a middle layer containing 20 to 70 weight % polyurethane and 80 to 30 weight % polycaprolactone, and two thinner outer layers containing a higher polycaprolactone content and having a lower weakening point than said middle layer.

As only the thinner outer layers of the core have to be heated above the temperature at which the thermoplastic material can stick through a coating, heating up time before use is shorter. As the middle layer has not to be brought at such high temperature as when the core is comprised of a single layer, the expansion of the core is also smaller. This is especially advantageous when the composite material is presented in the shape of a roll. Due to expansion when heating up, successive windings could be pressed so strongly together than they adhere strongly to each other.

The outer layers of the core may be comprised of pure polycaprolactone.

Usefully the outer layers of the core have each a thickness of about half the thickness of the middle layers.

The foam plastic coatings should withstand temperatures at which the core is softened or weakened. They are preferably made from a non-thermoplastic plastic, such as polyurethane.

In an advantageous embodiment of the invention, at least one layer of the core comprises 1 to 40 weight % of microspheres of non-metallic, heat-accumulating material.

In this form embodiment the composite material has moreover a slower cooling and is especially suited for heating-up in a micro-wave oven.

Especially suited are glass microspheres with a diameter between 20 and 800 micrometer.

Usefully a colouring agent can be added to the middle layer of the core.

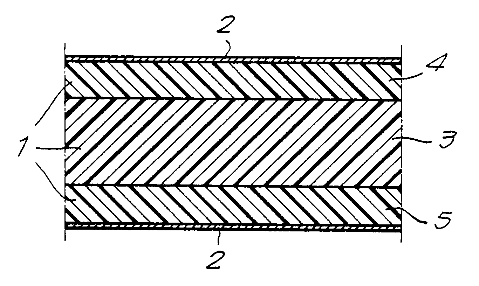

Other features and advantages of the invention will stand out from the following description of a composite material for medical or paramedical, particularly orthopaedic use, according to the invention. Said description is only given by way of example and does not limit the invention; the reference numerals pertain to the accompanying drawing.

The figure shows a cross-section of part of a composite material according to the invention.

The composite material as shown in the figure is comprised of a core 1 and of a foam plastic coating 2 on either side of said core. The core 1 is comprised of three layers : a middle layer 3 and two outer layers 4 and 5.

The middle layer 3 is made from a thermoplastic composition comprised of 20 to 70 weight % and preferably 50 to 60, for example 55 weight % polyurethane, and 80 to 30 weight % and preferably 50 to 40 weight % for example 45 weight % polycaprolactone.

The molecular weight of the polyurethane preferably lies between 10,000 and 100,000 and the molecular weight of the polycaprolactone preferably lies between 10,000 and 60,000 in particular preferably between 37,000 and 50,000.

Polyester polyurethanes are mostly suitable as polyurethane.

Caprolactone polyester polyurethane is particularly suitable, which polyurethane may be obtained by reacting isocyanate and polycaprolactone-based polyester.

Such a caprolactone polyester polyurethane is put on the market as a granulate by B.F. Goodrich Belgium under the name Estane R, type 5720.

The melting point of said polycaprolactone polyester polyurethane lies between 190 and 210 degrees Celsius. By adding the polycaprolactone, also preferably in granulate form, there is obtained a thermoplastic composition which is already distortable and kneadable at a temperature of 69 degrees Celsius and remains distortable by cooling down about 50 degrees Celsius.

At this temperature, a layer af said material can be stretched at least up to twenty times the original lenght thereof.

In cold condition, the thermoplastic composition is relatively rigid.

The outer layers 4 and 5 are from a thermoplastic composition similar to the composition of the middle layer 3, but have a higher caprolactone content and therefore a lower weakening point and a lower viscosity when heated than the middle layer 3. At the limit the outer layers 4 and 5 consist of 100% polycaprolactone.

The thickness of the outer layers 4 and 5 is advantageously about half the thickness of the middle layer 3.

The thickness of core 1 normally lies between 0.5 and 25 mm.

The coating 2 to the contrary are markedly thinner. They have a thickness outside the core 1 between 0.05 and 1.5 mm, and are from so-called soft plastic open cell foam.

When the composite material is presented in the shape of a roll, the maximum thickness of the foam coating is preferably of 0.6 mm and when the material is used as a small strip for winding around body parts the thickness of the foam coating is preferably not higher than 0.4 mm. The foam should have such open-cell structure that core material in softened condition can traverse it when pressure is excerted but also that the layer can be elastically deformed without tearing. When the composite material is heated up, it can be stretched up to four times its original lenght.

Suitable foam plastics for the coatings 2 are polyurethane, particularly polyester polyurethane and polyether foam.

For some applications, one or both coatings 2 may be provided with perforations (not shown) with a diameter of at least 0.5 mm and for example 2 mm. Such perforations are for example required when heating the composite material occurs in a warm water bath. The plastic foam absorbes water. Even after squeezing the water out of the plastic foam, there still remains a water fraction which opposes sticking. In the location of part at least of the perforations there is no water, and the plastic from core 1 of one layer can contact the plastic from the core 1 of an above-lying layer.

Cross-wise through core 1 and coating 2, smaller perforations with a diameter of at least 0.5 mm and preferably about 1 to 1.3 mm may be provided, so as not to hamper the skin breathing after applying the material. Said perforations may lie on rows crossing each other under 90 degrees and making an angle of 45 degrees with the transversal direction of the strip, plate or roll of composite material, at a distance of each other in the rows of 1.5 to 4 mm .

The fact that outer layers 4 and 5 have a lower weakening point results in the thermoplastic material of these relatively thin outer layers weakening more rapidly and having more rapidly a low viscosity when the composite material is heated up than when the whole core 1 has to be weakened. The thermoplastic material of these outer layers 4 and 5 is already able to traverse the foam coatings 2 when the middle layer 3 is still too rigid.

Not only the warming up time is shorter but the expansion of the composite material due to heating up is also smaller what is especially advantageous when the composite material is presented in the shape of a roll. Due to the expansion of the whole core 1 the successive windings would be pressed together and possibly stick to each other.

The middle layer 3 of the core 1 comprises besides 99 to 60 weight % of the thermoplastic composition as mentioned before, also 1 to 40 weight % of microspheres of non-metallic heat accumulating material. Particular suitable materials are ceramics, more particularly glass. The microspheres have a diameter between 50 micrometer and 800 micrometer, preferably about 140 micrometer. Such glass microspheres reduce costs. Moreover the microspheres increase the hardness of the layer 3 in cold condition. Strenght of the layer is improved and the layer 3 may be thinner also reducing costs.

A very important advantage of the microspheres is that due to their heat-accumulation, the cooling of the core 1, which is already relatively slow due to the coatings forming insulating layers, is much slower so that the time for applying the composite material on the body is much increased.

The microspheres have also the supplemental advantage to clean the extruder which is normally used for manufacturing the core 1 or the complete composite material.

Especially when warmed-up in a micro-wave oven the microspheres are heated and accumulate the heat. The heat is irradiated to the outer layers of the core which become quickly enough soft and nearly liquid to traverse the foam layers when portions of the composite materials are pressed together. When weakened or heated-up the thermoplastic material is transparent. In cold and hard condition it is rather opaque. It is therefore useful to add a colouring material to the middle layer 3 of the core 1. A soon as the outer layers 4 and 5 have been sufficiently weakened to permit adhesion they are transparent and the colour of the middle layer 3 which is not yet weakened and not transparent will become visible through the very thin foam coating 2.

As long as the colour of the layer 3 is visible the core 1 is not yet completely hardened. So it is possible to see when the composite material is completely cooled off. Warming up of the composite material, especially when it is in the shape of a roll, can quickly be performed in a micro-wave oven.

Due to the presence of coatings 2 of plastic foam, the composite material may be applied directly in distortable condition on the skin. It does not stick to hair and skin. It does not either leave visible fingerprints, so that throw-away gloves do not have to be used for the application. It does let X-rays well through.

Portions of the composite material 1,2 which are brought one against the other, for example the ends of a strip of such material, adhere in the plastic condition of core 1, sufficiently to one another to remain sticking to one another but not so strongly that they can not be released any more from one another. The core portions always adhere through two thin layers of coating 2. After some time however, under the action of the pressure from the above-lying material portion, the distortable plastic squeezes through the thin layers of coating 2 and after cooling, the portions strongly stick to one another. After being heated again, the portions may however be pulled away again from one another.

When applying the composite material, corrections may thus easily be made and a piece from the composite material may also be used anew a plurality of times.

Possible residues from the material are also not lost. A plurality of material layers may be laid over one another in plasticized condition.

As the coatings 2 form a thin insulating layer, the skin is subjected to little trouble from the relatively high temperature which is required to make the core 1 plastic and distortable.

The skin may still breathe even after application of the material.

Heating the material to plasticize the core preferably occurs in dry condition, for example by means of hot air, or in a micro-wave oven, although heating in a liquid is possible. In this latter case, the liquid has to be pressed out of the foam plastic coating before making use of the composite material.

The composite material is relatively homogeneous, does not crumble and is simple to work with, wear-resistant, withstands chemicals and ageing processes, and is impact-resistant.

The composite material is thus particularly suitable for replacing plaster bandages, splints or similar.

The invention will be illustrated more in detail by the following examples :

### Examples:

A three layer core 1 is co-extruded with on the outer sides a layer of soft open cell polyurethane. The middle layer of the core is a mixture of 40 weight % polycaprolactone and 60 weight % of polycaprolactone polyester polyurethane to which are added glass microspheres and a colouring agent.

The outer layers do not have a colouring agent or glass microspheres. They consist of a mixture of 50 weight % polycaprolactone and 50 weight % polycaprolactone polyester polyurethane.

### Example 1:

- middle layer :: 1 weight % glass microspheres with a diameter of 20 micrometer are added thickness : 0.2 mm.
- outer layers :: thickness : 0.1 mm.
- foam coatings :: thickness : 0.1 mm.

total thickness of composite layer : 0.5 mm.
heating time in a micro-wave oven of 600 W : 1.2 minutes
shape : strip or roll.
open time for applying the material : 10 minutes.

### Example 2:

Same as example 1 but microspheres have a diameter of 150 micrometer. The necessary heating time was 1.5 minutes.

### Example 3:

- middle layer :: 40 wt % glass microspheres with a diameter of 800 micrometer.
- thickness :: 20 mm.
- outer layers :: thickness : 9 mm.
- foam coatings :: thickness : 1.4 mm.

### Example 4:

middle layer : 20 wt % glass microspheres with a diameter of 140 micrometer, thickness : 2 mm.
outer layers : thickness : 0.9 mm.
foam coatings : thickness : 0.1 mm.
shape : strips, rolls or plates.

The above described composite material may be used in a lot of applications. The arrangement or applying thereof is quite easy. Portions from the material simply put on one another in plastic condition, do not stick or very slightly to one another, and it is but when such portions are pressed together that the fastening is obtained which, after becoming rigid, is very strong. As long as such cooling has not occured, the portions may still be loosened from one another.

The composite material is also very temperature resistant. Even should the material be excessively heated, for example to 150 degrees Celsius during thirty minutes, which is not excluded as heating occurs preferably not in a water bath but inside an oven, on a hot plate or in a hot air stream, the composite material does not lose in any way the properties thereof. It is only necessary to wait somewhat longer before the composite material can be used.

When heated in an oven or on a heating plate, the foam plastic prevents direct contact of the thermoplastic material with the oven wall or heating plate, limiting the temperature increase of the core.

The coatings 2 from foam plastic also insure anti-slip properties. Once a bandage or splint from said composite material has been laid on the body, the foam plastic opposes the sliding of the bandage or splint. This is mostly of importance when for example the material is arranged on moving limbs such as a foot for example.

The foam plastic is so thin that it does not accumulate dust or dirt. If necessary it can be cleaned with a brush. The coating prevent portions of the material undesirably sticking to each other or to the hands.

Thicker foam layers would prevent adhering together of overlapping portions of the composite material as the weakened thermoplastic core material would not be able to traverse the foam layers.

The invention is in no way limited to the above-described embodiments and within the scope of the described embodiments, many changes may be brought notably as regards the compositions, the imparted shapes, the size and the applications.

## Claims

1. Composite material for medical or paramedical use, particularly orthopaedic, which comprises a core (1) of a thermoplastic composition containing polycaprolactone and polyester and having a thickness between 0.05 and 25 mm, and on both sides thereof a coating of a soft resilient open cell foam plastic with a thickness outside the core (1) between 0.05 and 1.5 mm, characterized in that the core (1) is comprised of three layers: a middle layer (3) of a thermoplastic composition containing 20 to 70 weight-% polyurethane and 80 to 30 weight-% polycaprolactone and two thinner outer layers (4 and 5) containing a higher polycaprolactone content and having a lower weakening point than said middle layer (3).

2. Composite material according to claim 1, characterized in that the outer layers (4 and 5) of the core (1) are comprised of pure polycaprolactone.

3. Composite material according to 1, characterized in that the outer layers (4 and 5) of the core (1) are comprised of a higher polycaprolactone content than the middle layer, the remainder of the outer layers (4 and 5) being polyurethane.

4. Composite material according to any one of claims 1 to 3, characterized in that the outer layers (4 and 5) of the core (1) have each a thickness of about half the thickness of the middle layer (3).

5. Composite material according to any one of claims 1 to 4, characterized in that the thermoplastic composition of the middle layer (3) of the core (1) contains 20 to 70 weight-% caprolactone polyester polyurethane and 80 to 30 weight-% polycaprolactone.

6. Composite material according to any one of claims 1 to 5, characterized in that the coatings (2) of foam plastic are made from non-thermoplastic plastic.

7. Composite material according to any one of claims 1 to 6 characterized in that the coatings (2) of soft foam plastic are layers of polyurethane foam.

8. Composite material according to any one of claims 1 to 7, characterized in that at least one layer (3, 4 or 5) of the core (1) comprises 1 to 40 weight-% of microspheres of non-metallic, heat-accumulating material.

9. Composite material according to claim 8 characterized in that at least one layer (3, 4, 5) of the core (1) comprises about 20 weight-% of microspheres of non-metallic, heat-accumulating material.

10. Composite material according to claim 8 or 9, characterized in that the microspheres are glass microsperes.

11. Composite material according to any one of claims 8 to 10, characterized in that the microspheres have a diameter between 20 and 800 micrometer.

12. Composite material according to any one of claims 8 to 11, characterized in that only the middle layer (3) of the core (1) comprises the microspheres of non-metallic, heat-accummulating material.

13. Composite material according to any one of claims 1 to 12, characterized in that the middle layer (3) of the core (1) comprises a colouring agent.

14. Composite material according to any one of claims 1 to 13, characterized in that at least the coatings (2) are provided with regularly spaced perforations with a diameter of at least 0.5 mm.

15. Composite material according to any one of claims 1 to 14, characterized in that the coatings (2) of soft foam plastic on both sides of the core (1) have a thickness outside of the core between 0. 05 and 0. 8 mm.

16. Composite material according to claim 15, characterized in that the coatings (2) on both sides of the core (1) have a thickness of about 0.05 to 0.4 mm.

## Patentansprüche

1. Kompositmaterial für medizinische oder paramedizinische, insbesondere orthopädische, Verwendung, das einen Kern (1) aus einer thermoplastischen Verbindung beinhaltet, welche Polycaprolacton und Polyester enthält und eine Dicke zwischen 0,05 und 25 mm aufweist, sowie auf dessen beiden Seiten über eine Beschichtung (2) aus einem weichen, nachgiebigen, offenzelligen Schaumkunststoff mit einer Dicke zwischen 0,05 und 1,5 mm außerhalb des Kerns (1) verfügt, dadurch gekennzeichnet, daß der Kern (1) durch drei Schichten gebildet wird: eine mittlere Schicht (3) aus einer thermoplastischen Verbindung, die 20 bis 70 Gewichtsprozente Polyurethan und 80 bis 30 Gewichtsprozente Polycaprolacton enthält sowie zwei dünnere äußere Schichten (4 und 5), die einen höheren Polycaprolactongehalt haben und einen niedrigeren Weichpunkt als besagte Schicht (3) aufweisen.

2. Kompositmaterial nach Anspruch 1, dadurch gekennzeichnet, daß die äußeren Schichten (4 und 5) des Kerns (1) aus reinem Polycaprolacton bestehen.

3. Kompositmaterial nach Anspruch 1, dadurch gekennzeichnet, daß die äußeren Schichten (4 und 5) des Kerns (1) einen höheren Polycaprolactongehalt aufweisen als die mittlere Schicht, wobei der Rest der äußeren Schichten (4 und 5) Polyurethan ist.

4. Kompositmaterial nach einem der Ansprüche 1 bis 3, dadurch gekennzeichnet, daß die äußeren Schichten (4 und 5) des Kerns (1) jede eine Dicke von etwa der Hälfte der mittleren Schicht (3 ) aufweisen.

5. Kompositmaterial nach einem der Ansprüche 1 bis 4, dadurch gekennzeichnet, daß die thermoplastische Verbindung der mittleren Schicht (3) des Kerns (1) 20 bis 70 Gewichtsprozente Caprolacton-Polyester-Polyurethan und 80 bis 30 Gewichtsprozente Polycaprolacton enthält.

6. Kompositmaterial nach einem der Ansprüche 1 bis 5, dadurch gekennzeichnet, daß die Schaumkunststoff-Beschichtungen (2) aus nicht-thermoplastischem Kunststoff hergestellt sind.

7. Kompositmaterial nach einem der Ansprüche 1 bis 6, dadurch gekennzeichnet, daß die Beschichtungen (2) aus weichem Schaumkunststoff Schichten aus Polyurethanschaum sind.

8. Kompositmaterial nach einem der Ansprüche 1 bis 7, dadurch gekennzeichnet, daß zumindest eine Schicht (3, 4 oder 5) des Kerns (1) 1 bis 40 Gewichtsprozente von Mikrokügelchen aus nichtmetallischem, hitzespeicherndem Material enthält.

9. Kompositmaterial nach Anspruch 8, dadurch gekennzeichnet, daß zumindest eine Schicht (3, 4, 5) des Kerns (1) etwa 20 Gewichtsprozente an Mikrokügelchen aus nichtmetallischem, hitzespeicherndem Material enthält.

10. Kompositmaterial nach Anspruch 8 oder 9, dadurch gekennzeichnet, daß die Mikrokügelchen aus Glas bestehen.

11. Kompositmaterial nach einem der Ansprüche 8 bis 10, dadurch gekennzeichnet, daß die Mikrokügelchen einen Durchmesser zwischen 20 und 800 Mikrometer aufweisen.

12. Kompositmaterial nach einem der Ansprüche 8 bis 11, dadurch gekennzeichnet, daß nur die mittlere Schicht (3) des Kerns (1) die Mikrokügelchen aus nichtmetallischem, hitzespeicherndem Material enthält.

13. Kompositmaterial nach einem der Ansprüche 1 bis 12, dadurch gekennzeichnet, daß die mittlere Schicht (3) des Kerns (1) einen Farbzusatz enthält.

14. Kompositmaterial nach einem der Ansprüche 1 bis 13, dadurch gekennzeichnet, daß zumindest die Beschichtungen (2) mit regelmäßig angeordneten Perforationen mit einem Durchmesser von mindestens 0,5 mm versehen sind.

15. Kompositmaterial nach einem der Ansprüche 1 bis 14, dadurch gekennzeichnet, daß die Beschichtungen (2) aus weichem Schaumkunststoff auf beiden Seiten des Kerns (1) außerhalb des Kerns eine Dicke zwischen 0,05 und 0,8 mm aufweisen.

16. Kompositmaterial nach Anspruch 15, dadurch gekennzeichnet, daß die Beschichtungen (2) auf beiden Seiten des Kerns (1) eine Dicke von etwa 0,05 bis 0,4 mm aufweisen.

## Revendications

1. Matériau composite à usage médical ou paramédical, en particulier à usage orthopédique, qui comprend une âme (1) d'une composition thermoplastique contenant un polycaprolactone et un polyester et d'une épaisseur comprise entre 0,05 et 25 mm, présentant sur ses deux faces un revêtement (2) d'une mousse plastique à cellules ouvertes élastique et souple d'une épaisseur en dehors de l'âme (1) comprise entre 0,05 et 1,5 mm, caractérisé en ce que l'âme (1) est constituée de trois couches : une couche centrale (3) d'une composition thermoplastique contenant 20 à 70 % en poids de polyuréthane et 80 à 30 % en poids de polycaprolactone et deux couches externes plus minces (4 et 5) ayant une teneur supérieure en polycaprolactone et un point d'affaiblissement inférieur à celui de ladite couche centrale (3).

2. Matériau composite selon la revendication 1, caractérisé en ce que les couches externes (4 et 5) de l'âme (1) sont constituées de polycaprolactone pur.

3. Matériau composite selon la revendication 1, caractérisé en ce que les couches externes (4 et 5) de l'âme (1) ont une teneur en polycaprolactone plus élevée que la couche centrale, le restant des couches externes (4 et 5) étant constitué de polyuréthane.

4. Matériau composite selon l'une quelconque des revendications 1 à 3, caractérisé en ce que les couches externes (4 et 5) de l'âme (1) ont chacune une épaisseur d'environ la moitié de l'épaisseur de la couche centrale (3 ).

5. Matériau composite selon l'une quelconque des revendications 1 à 4, caractérisé en ce que la composition thermoplastique de la couche centrale (3) de l'âme (1) contient 20 à 70 % en poids de polyuréthane de polyester de caprolactone et 80 à 30 % poids de polycaprolactone.

6. Matériau composite selon l'une quelconque des revendications 1 à 5, caractérisé en ce que les revêtements (2) de mousse plastique sont constitués d'une matière plastique non thermoplastique.

7. Matériau composite selon l'une quelconque des revendications 1 à 6, caractérisé en ce que les revêtements (2) de mousse plastique souple sont des couches de mousse de polyuréthane.

8. Matériau composite selon l'une quelconque des revendications 1 à 7, caractérisé en ce qu'au moins une couche (3, 4 ou 5) de l'âme (1) comprend 1 à 40 % en poids de microsphères de matériau non métallique accumulant la chaleur.

9. Matériau composite selon la revendication 8, caractérisé en ce qu'au moins une couche (3, 4 ou 5) de l'âme (1) comprend environ 20 % en poids de microsphères de matériau non métallique accumulant la chaleur.

10. Matériau composite selon la revendication 8 ou 9 , caractérisé en ce que les microsphères sont des microsphères de verre.

11. Matériau composite selon l'une quelconque des revendications 8 à 10, caractérisé en ce que les microsphères ont un diamètre compris entre 20 et 800 micromètres.

12. Matériau composite selon l'une quelconque des revendications 8 à 11, caractérisé en ce que seule la partie centrale (3) de l'âme (1) contient les microsphères de matériau non métallique accumulant la chaleur.

13. Matériau composite selon l'une quelconque des revendications 1 à 12, caractérisé en ce que la couche centrale (3) de l'âme (1) comprend un agent colorant.

14. Matériau composite selon l'une quelconque des revendications 1 à 13, caractérisé en ce qu'au moins les revêtements (2) sont pourvus de perforations régulièrement espacées d'un diamètre d'au moins 0,5 mm.

15. Matériau composite selon l'une quelconque des revendications 1 à 14, caractérisé en ce que les revêtements (2) de mousse plastique souple sur les deux faces de l'âme (1) ont une épaisseur, en dehors de l'âme, comprise entre 0,05 et 0,8 mm.

16. Matériau composite selon la revendication 15, caractérisé en ce que les revêtements (2) sur les deux faces de l'âme (1) ont une épaisseur d'environ 0,05 à 0,4 mm.
